# EUROPEAN PATENT APPLICATION

(11) **EP 3 932 429 A1**
(43) Date of publication of application: **05.01.2022**
(21) Application number: 20761500.6
(22) Date of filing: 27.02.2020
(51) Int. Cl.: A61K 45/00, A61P 35/00, A61P 35/02, A61P 43/00, A61K 31/505

(54) **COMBINATIONAL MEDICATION**

(30) Priority: 28.02.2019 JP 2019035668
(71) Applicant: FUJIFILM Corporation, Tokyo 106-8620 (JP)
(72) Inventor: MURAO Hidetoshi, Ashigarakami-gun, Kanagawa 258-8577 (JP); OGURA Hayato, Ashigarakami-gun, Kanagawa 258-8577 (JP); SAITO Koichi, Ashigarakami-gun, Kanagawa 258-8577 (JP); HAGIWARA Shinji, Ashigarakami-gun, Kanagawa 258-8577 (JP); YAMAURA Takeshi, Ashigarakami-gun, Kanagawa 258-8577 (JP); NAKATANI Toshiyuki, Ashigarakami-gun, Kanagawa 258-8577 (JP); KIYOI Hitoshi, Nagoya-shi, Aichi 464-8601 (JP); ISHIKAWA Yuichi, Nagoya-shi, Aichi 464-8601 (JP)
(74) Representative: Hoffmann Eitle
(86) International application number: PCT/JP2020/008063
(87) International publication number: WO 2020/175629

(57) **Abstract**

An object of the present invention is to provide a combination medicine that exhibits a practical curing effect on a tumor such as acute myeloid leukemia. According to the present invention, there is provided a combination medicine, which contains a compound represented by General Formula [1] specified in the present specification, such as (S,E)-N-(1-((5-(2-((4-cyanophenyl)amino)-4-(propylamino)pyrimidin-5-yl)-4-pentyn-1-yl)ami no)-1-oxopropan-2-yl)-4-(dimethylamino)-N-methyl-2-butenamide or a salt thereof, and at least one selected from the group consisting of a BCL-2 inhibitor and a pyrimidine antimetabolite.

## Description

### Technical Field

The present invention relates to a combination medicine useful for the treatment of hematological cancer.

### Background Art

A nitrogen-containing heterocyclic compound having an excellent Fms-like tyrosine kinase 3 (FLT3) inhibitory activity and being useful as a drug substance for pharmaceuticals has been reported (Patent Documents 1 and 2). In addition, a pharmaceutical composition for treating FLT3 mutation-positive cancer, which contains the above nitrogen-containing heterocyclic compound, has been reported (Patent Document 3). Further, methods for producing the nitrogen-containing heterocyclic compound and an intermediate thereof have been reported (Patent Document 4). Hereinafter, the compound represented by General Formula [1] disclosed in Patent Document 3 or a salt thereof may be simply referred to as Compound A.

Patent Document 5 describes a combination therapy of an imidazolothiazole compound, which is an FLT3 inhibitor, with azacytidine or cytarabine.

### Prior Art Documents

### Patent Documents

Patent Document 1: WO2013/157540A
Patent Document 2: WO2015/056683A
Patent Document 3: WO2016/027904A
Patent Document 4: WO2017/010535A
Patent Document 5: WO2010/111172A

### SUMMARY OF THE INVENTION

FLT3 plays an important role in the proliferation and the differentiation of hematopoietic cells. In the normal bone marrow, expression of FLT3 is observed in hematopoietic stem cells, progenitor cells, and the like; however, FLT3 is overexpressed or FLT3 is mutated in hematological cancer, which results in the activation of the FLT3 signaling pathway and contributes to the proliferation and malignant transformation of cancer. A new curing method for such diseases is desired.

An object of the present invention is to provide a combination medicine that exhibits a practical curing effect on a tumor such as acute myeloid leukemia.

As a result of diligent studies to solve the above problems, the inventors of the present invention have found that in a case where Compound A is used in combination with at least one selected from the group consisting of a BCL-2 inhibitor and a pyrimidine antimetabolite, it is possible to achieve a synergistic effect on the proliferation inhibitory activity against a leukemia cell line MV-4-11 and a leukemia patient-derived cell, which are FLT3-ITD mutation-positive. The present invention has been completed based on the above findings.

That is, the present invention provides the followings.
<1> A combination medicine comprising:
   a compound represented by General Formula [1] or a salt thereof; and
   at least one selected from the group consisting of a BCL-2 inhibitor and a pyrimidine antimetabolite, 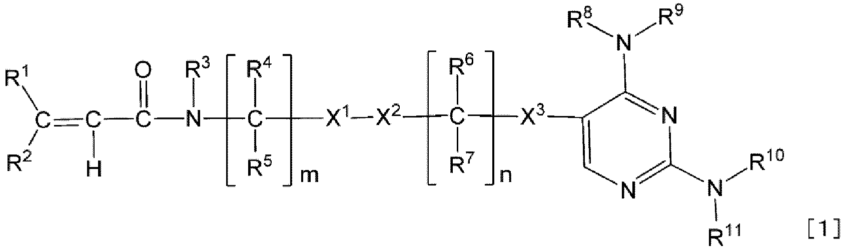
   in the formula,
      R¹ represents a hydrogen atom or a C₁₋₆ alkyl group which may be substituted,
      R² represents a hydrogen atom, a C₁₋₆ alkyl group which may be substituted, a C₂₋₆ alkenyl group which may be substituted, or a C₂₋₆ alkynyl group which may be substituted,
      R³ represents a hydrogen atom, a C₁₋₆ alkyl group which may be substituted, a C₂₋₆ alkenyl group which may be substituted, or a C₂₋₆ alkynyl group which may be substituted,
      m represents an integer of 1 to 3,
      m pieces of R⁴'s may be the same or different from each other and represent a hydrogen atom or a C₁₋₆ alkyl group which may be substituted, where one R⁴ selected from the m pieces of R⁴'s may be combined together with R³ to form a C₁₋₆ alkylene group which may be substituted,
      m pieces of R⁵'s may be the same or different from each other and represent a hydrogen atom, a C₁₋₆ alkyl group which may be substituted, a C₂₋₆ alkenyl group which may be substituted, or a C₂₋₆ alkynyl group which may be substituted,
      X¹ represents an oxygen atom, N(R²⁰) (in the formula, R²⁰ represents a hydrogen atom, a C₁₋₆ alkyl group which may be substituted, a C₂₋₆ alkenyl group which may be substituted, or a C₂₋₆ alkynyl group which may be substituted), C(=O), C(=O)-N(R²⁰) (in the formula, R²⁰ has the same meaning as above), or a bond,
      X² represents a C₁₋₆ alkylene group which may be substituted, a divalent alicyclic hydrocarbon group which may be substituted, or a divalent aromatic hydrocarbon group which may be substituted,
      n represents an integer of 0 to 3,
      n pieces of R⁶'s may be the same or different from each other and represent a hydrogen atom or a C₁₋₆ alkyl group which may be substituted,
      n pieces of R⁷'s may be the same or different from each other and represent a hydrogen atom or a C₁₋₆ alkyl group which may be substituted,
      X³ represents a C₁₋₆ alkylene group which may be substituted, a C₂₋₆ alkenylene group which may be substituted, a C₂₋₆ alkynylene group which may be substituted, or N(R²⁰)-C(=O) (in the formula, R²⁰ has the same meaning as the above),
      R⁸ represents a hydrogen atom, a C₁₋₆ alkyl group which may be substituted, a C₂₋₆ alkenyl group which may be substituted, or a C₂₋₆ alkynyl group which may be substituted,
      R⁹ represents a C₁₋₆ alkyl group which may be substituted, a C₂₋₆ alkenyl group which may be substituted, a C₂₋₆ alkynyl group which may be substituted, or a C₃₋₈ cycloalkyl group which may be substituted,
      R⁸ and R⁹ may be combined together with a nitrogen atom to which R⁸ and R⁹ are bonded, to form a cyclic amino group which may be substituted,
      R¹⁰ represents a hydrogen atom, a C₁₋₆ alkyl group which may be substituted, a C₂₋₆ alkenyl group which may be substituted, or a C₂₋₆ alkynyl group which may be substituted, and
      R¹¹ represents a C₁₋₆ alkyl group which may be substituted, a C₂₋₆ alkenyl group which may be substituted, a C₂₋₆ alkynyl group which may be substituted, a C₃₋₈ cycloalkyl group which may be substituted, an aryl group which may be substituted, or a heterocyclic group which may be substituted.
<2> The combination medicine according to <1>, in which R¹⁰ is a hydrogen atom, and
   X¹ is C(=O)-N(R²⁰) (in the formula, R²⁰ represents a hydrogen atom, a C₁₋₆ alkyl group which may be substituted, a C₂₋₆ alkenyl group which may be substituted, or a C₂₋₆ alkynyl group which may be substituted).
<3> The combination medicine according to <1> or <2>, in which X³ is a C₂₋₆ alkynylene group which may be substituted.
<4> The combination medicine according to any one of <1> to <3>, in which the compound represented by General Formula [1] is (S,E)-N-(1-((5-(2-((4-cyanophenyl)amino)-4-(propylamino)pyrimidin-5-yl)-4-pentyn-1-yl)ami no)-1-oxopropan-2-yl)-4-(dimethylamino)-N-methyl-2-butenamide.
<5> The combination medicine according to any one of <1> to <4>, in which a daily dose of the compound represented by General Formula [1] or the salt thereof is 10 to 450 mg.
<6> The combination medicine according to any one of <1> to <5>, in which the compound represented by General Formula [1] or the salt thereof and at least one selected from the group consisting of the BCL-2 inhibitor and a pyrimidine antimetabolite are provided as the same composition or as separate compositions.
<7> The combination medicine according to any one of <1> to <6>, in which the compound represented by General Formula [1] or the salt thereof is orally administered, and the pyrimidine antimetabolite is intravenously administered by drip infusion or a BCL-2 inhibitor is orally administered.
<8> The combination medicine according to any one of <1> to <7>, in which the combination medicine contains, as the pyrimidine antimetabolite, a compound selected from the group consisting of azacytidine, decitabine, guadecitabine, cytarabine, and gemcitabine, or a salt or hydrate thereof.
<9> The combination medicine according to any one of <1> to <8>, in which the combination medicine contains venetoclax or a salt thereof as the BCL-2 inhibitor.
<10> The combination medicine according to any one of <1> to <8>, in which the combination medicine contains, as the pyrimidine antimetabolite, decitabine or a salt or hydrate thereof, and a daily dose of the decitabine or a salt or hydrate thereof is 1 to 80 mg/m2.
<11> The combination medicine according to any one of <1> to <6> and <9>, in which the compound represented by General Formula [1] or a salt thereof and the BCL-2 inhibitor are provided as a same composition.
<12> The combination medicine according to <11>, in which the combination medicine contains 0.3% to 50% by mass of the compound represented by General Formula [1] or the salt thereof and 40% to 99.7% by mass of the BCL-2 inhibitor with respect to a total amount of the combination medicine.
<13> The combination medicine according to <11> or <12>, in which the combination medicine is a tablet.
<14> The combination medicine according to any one of <1> to <13>, in which the combination medicine is used for treatment of hematological cancer.
<15> The combination medicine according to any one of <1> to <14>, in which the combination medicine is used for treatment of acute myeloid leukemia.

(A) A method for using Compound A and at least one selected from the group consisting of a BCL-2 inhibitor and a pyrimidine antimetabolite to treat a tumor, the method including administering Compound A and at least one selected from the group consisting of a BCL-2 inhibitor and a pyrimidine antimetabolite to a subject (a mammal, including a human) in need of tumor treatment.
(B) A method for treating a tumor, including administering Compound A and at least one selected from the group consisting of a BCL-2 inhibitor and a pyrimidine antimetabolite to a subject (a mammal, including a human) in need of tumor treatment.
(C) Use of a combination of Compound A and at least one selected from the group consisting of a BCL-2 inhibitor and a pyrimidine antimetabolite, for producing an anti-tumor agent.
(D) A combination of Compound A and at least one selected from the group consisting of a BCL-2 inhibitor and a pyrimidine antimetabolite, for use in the curing of a tumor.

A combination of Compound A and at least one selected from the group consisting of a BCL-2 inhibitor and a pyrimidine antimetabolite exhibits a curing effect on a tumor such as acute myeloid leukemia.

### DESCRIPTION OF THE PREFERRED EMBODIMENTS

In the present invention, the range represented by "to" includes the values at both ends thereof unless otherwise specified.

The "subject" is a mammal such as a human, a mouse, a monkey, or a domestic animal requiring prevention or curing therefor, and preferably a human requiring prevention or curing therefor.

The "prevention" means the inhibition of the onset of a disease, the reduction of the risk of the onset of a disease, or the delay of onset of a disease.

The "curing" means the amelioration or the suppression (the maintenance or delay) of the progression of a disease or a state of interest.

The "treatment" means the prevention or the curing of various diseases.

The "tumor" means a benign or malignant tumor.

The "benign tumor" means a tumor in which the morphology of a tumor cell and the sequence of the tumor cell are similar to those of the normal cell from which the tumor cell is derived and which is not invasive or metastatic.

The malignant tumor means a tumor in which the morphology of a tumor cell and the sequence of the tumor cell are different from those of the normal cell from which the tumor cell is derived and which is invasive or metastatic.

The "dose per administration" means a dose of Compound A per administration for a human. The human is preferably an adult.

Examples of the halogen atom include a fluorine atom, a chlorine atom, a bromine atom, and an iodine atom.

Examples of the C₁₋₆ alkyl group include linear or branched C₁₋₆ alkyl groups such as a methyl group, an ethyl group, a propyl group, an isopropyl group, a butyl group, a sec-butyl group, an isobutyl group, a tert-butyl group, a pentyl group, an isopentyl group, and a hexyl group.

Examples of the C₁₋₃ alkyl group include a methyl group, an ethyl group, a propyl group, and an isopropyl group.

Examples of the C₂₋₆ alkenyl group include linear or branched C₂₋₆ alkenyl groups such as a vinyl group, an allyl group, a propenyl group, an isopropenyl group, a butenyl group, an isobutenyl group, a 1,3-butadienyl group, a pentenyl group, and a hexenyl group.

Examples of the C₂₋₆ alkynyl group include linear or branched C₂₋₆ alkynyl groups such as an ethynyl group, a propynyl group, a butynyl group, a pentynyl group, and a hexynyl group.

Examples of the C₃₋₈ cycloalkyl group include C₃₋₈ cycloalkyl groups such as a cyclopropyl group, a cyclobutyl group, a cyclopentyl group, and a cyclohexyl group.

Examples of the aryl group include a phenyl group and a naphthyl group.

Examples of the aryl C₁₋₆ alkyl group include aryl C₁₋₆ alkyl groups such as a benzyl group, a diphenylmethyl group, a trityl group, a phenethyl group, and a naphthylmethyl group.

Examples of the C₁₋₆ alkoxy group include linear, cyclic, or branched C₁₋₆ alkyloxy groups such as a methoxy group, an ethoxy group, a propoxy group, an isopropoxy group, a cyclopropoxy group, a butoxy group, an isobutoxy group, a sec-butoxy group, a tert-butoxy group, a cyclobutoxy group, a pentyloxy group, and hexyloxy group.

Examples of the C₁₋₃ alkoxy group include a methoxy group, an ethoxy group, a propoxy group, and an isopropoxy group.

Examples of the C₁₋₆ alkoxy C₁₋₆ alkyl group include C₁₋₆ alkyloxy C₁₋₆ alkyl groups such as a methoxymethyl group and an 1-ethoxyethyl group.

Examples of the aryl C₁₋₆ alkoxy C₁₋₆ alkyl group include aryl C₁₋₆ alkyloxy C₁₋₆ alkyl groups such as a benzyloxymethyl group and a phenethyloxymethyl group.

Examples of the C₂₋₆ alkanoyl group include linear or branched C₂₋₆ alkanoyl groups such as an acetyl group, a propionyl group, a valeryl group, an isovaleryl group, and a pivaloyl group.

Examples of the aroyl group include a benzoyl group and a naphthoyl group.

Examples of the heterocyclic carbonyl group include a nicotinoyl group, a thenoyl group, a pyrrolidinocarbonyl group, and a furoyl group.

Examples of the (a-substituted) aminoacetyl group include an (a-substituted) aminoacetyl group, the N-terminal of which may be protected and which is derived from an amino acid (examples of the amino acid include glycine, alanine, valine, leucine, isoleucine, serine, threonine, cysteine, methionine, aspartic acid, glutamic acid, asparagine, glutamine, arginine, lysine, histidine, hydroxylysine, phenylalanine, tyrosine, tryptophan, proline, and hydroxyproline).

Examples of the acyl group include a formyl group, a succinyl group, a glutaryl group, a maleoyl group, a phthaloyl group, a C₂₋₆ alkanoyl group, an aroyl group, a heterocyclic carbonyl group, and an (a-substituted) aminoacetyl group.

Examples of the acyl C₁₋₆ alkyl group include acyl C₁₋₆ alkyl groups such as an acetylmethyl group, a benzoylmethyl group, and a 1-benzoylethyl group.

Examples of the acyloxy C₁₋₆ alkyl group include acyloxy C₁₋₆ alkyl groups such as an acetoxymethyl group, a propionyloxymethyl group, a pivaloyloxymethyl group, a benzoyloxymethyl group, and a 1-(benzoyloxy)ethyl group.

Examples of the C₁₋₆ alkoxycarbonyl group include linear or branched C₁₋₆ alkyloxycarbonyl groups such as a methoxycarbonyl group, an ethoxycarbonyl group, an isopropoxycarbonyl group, a tert-butoxycarbonyl group, and a 1,1-dimethylpropoxycarbonyl group.

Examples of the aryl C₁₋₆ alkoxycarbonyl group include aryl C₁₋₆ alkyloxycarbonyl groups such as a benzyloxycarbonyl group and phenethyloxycarbonyl group.

Examples of the aryloxycarbonyl group include a phenyloxycarbonyl group and a naphthyloxycarbonyl group.

Examples of the C₁₋₆ alkylamino group include linear or branched C₁₋₆ alkylamino groups such as a methylamino group, an ethylamino group, a propylamino group, an isopropylamino group, a butylamino group, a sec-butylamino group, a tert-butylamino group, a pentylamino group, and a hexylamino group.

Examples of the di(C₁₋₆ alkyl) amino group include linear or branched di(C₁₋₆ alkyl) amino groups such as a dimethylamino group, a diethylamino group, a dipropylamino group, a diisopropylamino group, a dibutylamino group, a di (tert-butyl) amino group, a dipentylamino group, a dihexylamino group, an (ethyl)(methyl) amino group, and a (methyl)(propyl) amino group.

Examples of the di(C₁₋₃ alkyl) amino group include linear or branched di(C₁₋₃ alkyl) amino groups such as a dimethylamino group, a diethylamino group, a dipropylamino group, a diisopropylamino group, an (ethyl)(methyl) amino group, and a (methyl)(propyl) amino group.

Examples of the C₁₋₆ alkylsulfonyl group include C₁₋₆ alkylsulfonyl groups such as a methylsulfonyl group, an ethylsulfonyl group, and a propylsulfonyl group.

Examples of the arylsulfonyl group include a benzenesulfonyl group, a p-toluenesulfonyl group, and a naphthalenesulfonyl group.

Examples of the C₁₋₆ alkylsulfonyloxy group include C₁₋₆ alkylsulfonyloxy groups such as a methylsulfonyloxy group and an ethylsulfonyloxy group.

Examples of the arylsulfonyloxy group include a benzenesulfonyloxy group and a p-toluenesulfonyloxy group.

Examples of the cyclic amino group include a cyclic amino group which contains one or more nitrogen atoms and may further contain one or more oxygen atoms or sulfur atoms as a heteroatom constituting a ring of a group such as an azetidinyl group, a pyrrolidinyl group, a pyrrolinyl group, a pyrrolyl group, a piperidinyl group, a tetrahydropyridyl group, a homopiperidinyl group, an imidazolidinyl group, an imidazolinyl group, an imidazolyl group, a pyrazolydinyl group, a pyrazolinyl group, a pyrazolyl group, a piperazinyl group, a homopiperazinyl group, a triazolyl group, a tetrazolyl group, a morpholinyl group, a thiomorpholinyl group, a tetrahydroquinolinyl group, a tetrahydroisoquinolinyl group, or a quinuclidinyl group.

Examples of the monocyclic nitrogen-containing heterocyclic group include a monocyclic nitrogen-containing heterocyclic group which contains only a nitrogen atom as a heteroatom constituting a ring of a group such as an azetidinyl group, a pyrrolidinyl group, a pyrrolinyl group, a pyrrolyl group, a piperidyl group, a tetrahydropyridyl group, a pyridyl group, a homopiperidinyl group, an octahydroazosinyl group, an imidazolidinyl group, an imidazolinyl group, an imidazolyl group, a pyrazolydinyl group, a pyrazolinyl group, a pyrazolyl group, a piperazinyl group, a pyrazinyl group, a pyridazinylgroup, a pyrimidinyl group, a homopiperazinyl group, a triazolyl group, or a tetrazolyl group.

Examples of the monocyclic oxygen-containing heterocyclic group include a tetrahydrofuranyl group, a furanyl group, a tetrahydropyranyl group, and a pyranyl group.

Examples of the monocyclic sulfur-containing heterocyclic group include a thienyl group.

Examples of the monocyclic nitrogen-containing and the oxygen-containing heterocyclic group include a monocyclic nitrogen-containing and oxygen-containing heterocyclic group containing only a nitrogen atom and an oxygen atom as a heteroatom constituting a ring of a group such as an oxazolyl group, an isoxazolyl group, an oxadiazolyl group, or a morpholinyl group.

Examples of the monocyclic nitrogen-containing and sulfur-containing heterocyclic group include a monocyclic nitrogen-containing and sulfur-containing heterocyclic group containing only a nitrogen atom and a sulfur atom as a heteroatom constituting a ring of a group such as a thiazolyl group, an isothiazolyl group, a thiadiazolyl group, a thiomorpholinyl group, a 1-oxidothiomorpholinyl group, or a 1,1-dioxidothiomorpholinyl group.

Examples of the monocyclic heterocyclic group include a monocyclic nitrogen-containing heterocyclic group, a monocyclic oxygen-containing heterocyclic group, a monocyclic sulfur-containing heterocyclic group, a monocyclic nitrogen-containing and oxygen-containing heterocyclic group, and a monocyclic nitrogen-containing and sulfur-containing heterocyclic group.

Examples of the bicyclic nitrogen-containing heterocyclic group include a bicyclic nitrogen-containing heterocyclic group containing only a nitrogen atom as a heteroatom constituting a ring of a group such as an indolinyl group, an indolyl group, an isoindolinyl group, an isoindolyl group, a benzimidazolyl group, indazolyl group, a benzotriazolyl group, a pyrazolopyridinyl group, a tetrahydroquinolinyl group, a quinolyl group, a tetrahydroisoquinolinyl group, an isoquinolinyl group, a quinolizinyl group, a cinnolinyl group, a phthalazinyl group, a quinazolinyl group, a dihydroquinoxalinyl group, a quinoxalinyl group, a naphthyridinyl group, a purinyl group, a pteridinyl group, or a quinuclidinyl group.

Examples of the bicyclic oxygen-containing heterocyclic group include a bicyclic oxygen-containing heterocyclic group containing only an oxygen atom as a heteroatom constituting a ring of a group such as a 2,3-dihydrobenzofuranyl group, a benzofuranyl group, an isobenzofuranyl group, a chromanyl group, a chromenyl group, an isochromanyl group, a 1,3-benzodioxolyl group, a 1,3-benzodioxanyl group, or a 1,4-benzodioxanyl group.

Examples of the bicyclic sulfur-containing heterocyclic group include a bicyclic sulfur-containing heterocyclic group containing only a sulfur atom as a heteroatom constituting a ring of a group such as a 2,3-dihydrobenzothienyl group or a benzothienyl group.

Examples of the bicyclic nitrogen-containing and oxygen-containing heterocyclic group include a bicyclic nitrogen-containing and oxygen-containing heterocyclic group containing only a nitrogen atom and an oxygen atom as heteroatoms constituting a ring of a group such as a benzoxazolyl group, a benzisoxazolyl group, a benzoxadiazolyl group, a benzomorpholinyl group, a dihydropyranopyridyl group, a dihydrodioxynopyridyl group, or a dihydropyridooxadinyl group.

Examples of the bicyclic nitrogen-containing and sulfur-containing heterocyclic group include a bicyclic nitrogen-containing and sulfur-containing heterocyclic group containing a nitrogen atom and a sulfur atom as heteroatoms constituting a ring of a group such as a benzothiazolyl group, a benzisothiazolyl group, or a benzothiadiazolyl group.

Examples of the bicyclic heterocyclic group include a bicyclic nitrogen-containing heterocyclic group, a bicyclic oxygen-containing heterocyclic group, a bicyclic sulfur-containing heterocyclic group, a bicyclic nitrogen-containing and oxygen-containing heterocyclic group, and a bicyclic nitrogen-containing and sulfur-containing heterocyclic group.

Examples of the heterocyclic group include a monocyclic heterocyclic group and a bicyclic heterocyclic group.

Examples of the C₁₋₆ alkylene group include linear or branched C₁₋₆ alkylene groups such as a methylene group, an ethylene group, a propylene group, a butylene group, and a hexylene group.

Examples of the C₁₋₃ alkylene group include a methylene group, an ethylene group, and a propylene group.

Examples of the C₂₋₆ alkenylene group include linear or branched C₂₋₆ alkenylene groups such as a vinylene group, a propenylene group, a butenylene, and a pentenylene group.

Examples of the C₂₋₆ alkynylene group include linear or branched C₂₋₆ alkynylene groups such as an ethynylene group, a propynylene group, a butynylene group, and a pentynylene group.

Examples of the divalent alicyclic hydrocarbon group include a group formed by removing two hydrogen atoms from an alicyclic hydrocarbon ring, such as a 1,2-cyclobutylene group, a 1,3-cyclobutylene group, a 1,2-cyclopentylene group, a 1,3-cyclopentylene group, a 1,2-cyclohexylene group, a 1,3-cyclohexylene group, a 1,4-cyclohexylene group, a bicyclo(3.2.1) octylene group, a bicyclo(2.2.0) hexylene group, or a bicyclo(5.2.0) nonylene group.

Examples of the divalent aromatic hydrocarbon group include a group formed by removing two hydrogen atoms from an aromatic hydrocarbon ring, such as a phenylene group, an indenylene group, a naphthylene group, an FLuorenylene group, a phenanthrenylene group, anthrylene group, or a pyrenylene group.

Examples of the silyl group include a trimethylsilyl group, a triethylsilyl group, and tributylsilyl group.

The amino protecting group includes all groups that can be used as the typical protecting group for an amino group, and examples thereof include groups described in T. W. Greene et al., Protective Groups in Organic Synthesis, 4th Edition, pp. 696 to 926, 2007, John Wiley & Sons Inc. Specific examples thereof include an aryl C₁₋₆ alkyl group, a C₁₋₆ alkoxy C₁₋₆ alkyl group, an acyl group, a C₁₋₆ alkoxycarbonyl group, an aryl C₁₋₆ alkoxycarbonyl group, an aryloxycarbonyl group, a C₁₋₆ alkylsulfonyl group, an arylsulfonyl group, and a silyl group.

The imino protecting group includes all groups that can be used as the typical protecting group for an imino group, and examples thereof include groups described in T. W. Greene et al., Protective Groups in Organic Synthesis, 4th Edition, pp. 696 to 868, 2007, John Wiley & Sons Inc. Specific examples thereof include an aryl C₁₋₆ alkyl group, a C₁₋₆ alkoxy C₁₋₆ alkyl group, an acyl group, a C₁₋₆ alkoxycarbonyl group, an aryl C₁₋₆ alkoxycarbonyl group, an aryloxycarbonyl group, a C₁₋₆ alkylsulfonyl group, an arylsulfonyl group, and a silyl group.

The hydroxyl protecting group includes all groups that can be used as the typical protecting group for a hydroxyl group, and examples thereof include groups described in T. W. Greene et al., Protective Groups in Organic Synthesis, 4th Edition, pp. 16 to 299, 2007, John Wiley & Sons Inc. Specific examples thereof include a C₁₋₆ alkyl group, a C₂₋₆ alkenyl group, an aryl C₁₋₆ alkyl group, a C₁₋₆ alkoxy C₁₋₆ alkyl group, an aryl C₁₋₆ alkoxy C_{1- 6} alkyl group, an acyl groups, a C₁₋₆ alkoxycarbonyl group, an aryl C₁₋₆ alkoxycarbonyl group, a C₁₋₆ alkylsulfonyl group, an arylsulfonyl group, a silyl group, a tetrahydrofuranyl group, and a tetrahydropyranyl groups.

The carboxyl protecting group includes all groups that can be used as the typical protecting group for a carboxyl group, and examples thereof include groups described in T. W. Greene et al., Protective Groups in Organic Synthesis, 4th Edition, pp. 533 to 643, 2007, John Wiley & Sons Inc. Specific examples thereof include a C₁₋₆ alkyl group, a C₂₋₆ alkenyl group, an aryl group, an aryl C₁₋₆ alkyl group, a C₁₋₆ alkoxy C₁₋₆ alkyl group, an aryl C₁₋₆ alkoxy C₁₋₆ alkyl group, an acyl C₁₋₆ alkyl group, an acyloxy C₁₋₆ alkyl group, and a silyl group.

### <Compound of General Formula [1] and salt thereof>

Compound A in the present invention is a compound represented by General Formula [1] and a salt thereof.

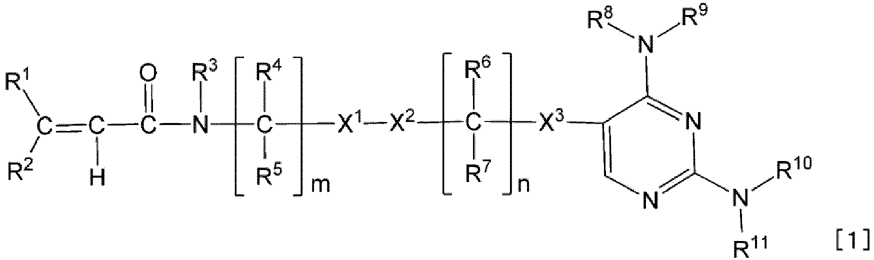

(In the formula, R¹, R², R³, R⁴, R⁵, R⁶, R⁷, R⁸, R⁹, R¹⁰, R¹¹, X¹, X², X³, m, and n have the same meanings as those described above.)

R¹ is a hydrogen atom or a C₁₋₆ alkyl group which may be substituted and preferably a hydrogen atom.

In any case where other substituents are any substituents, the C₁₋₆ alkyl group as R¹ may be substituted with one or more groups selected from a halogen atom, a cyano group, an amino group which may be protected, and a hydroxyl group which may be protected.

The C₁₋₆ alkyl group of the C₁₋₆ alkyl group which may be substituted, as R¹, is preferably a C₁₋₃ alkyl group.

R² is a hydrogen atom, a C₁₋₆ alkyl group which may be substituted, a C₂₋₆ alkenyl group which may be substituted, or a C₂₋₆ alkynyl group which may be substituted, preferably a hydrogen atom or a C₁₋₆ alkyl group which may be substituted, and more preferably a C₁₋₆ alkyl group which may be substituted.

In any case where other substituents are any substituents, the C₁₋₆ alkyl group, the C₂₋₆ alkenyl group, or the C₂₋₆ alkynyl group, as R², may be substituted with one or more groups selected from a C₁₋₆ alkylamino group which may be substituted with one or more groups selected from the substituent group A, a di(C₁₋₆ alkyl) amino group which may be substituted with one or more groups selected from the substituent group A, and a heterocyclic group which may be substituted with one or more groups selected from the substituent group A.

The substituent group A: a halogen atom, a cyano group, an amino group which may be protected, a hydroxyl group which may be protected, a C₁₋₆ alkyl group which may be substituted with one or more groups selected from the substituent group B, a C₃₋₈ cycloalkyl group which may be substituted with one or more groups selected from the substituent group B, an aryl group which may be substituted with one or more groups selected from the substituent group B, a C₁₋₆ alkoxy group which may be substituted with one or more groups selected from the substituent group B, a C₁₋₆ alkylamino group which may be substituted with one or more groups selected from the substituent group B, a di(C₁₋₆ alkyl) amino group which may be substituted with one or more groups selected from the substituent group B, or a heterocyclic group which may be substituted with one or more groups selected from the substituent group B, and an oxo group.

The substituent group B: a halogen atom, a cyano group, an amino group which may be protected, a hydroxyl group which may be protected, a C₁₋₆ alkyl group which may be substituted with a halogen atom or a hydroxyl group, a C₁₋₆ alkoxy group which may be substituted with a halogen atom or a hydroxyl group, an aryl group, a heterocyclic group, and an oxo group.

The C₁₋₆ alkyl group which may be substituted, as R², is preferably a C₁₋₆ alkyl group which is substituted with a di(C₁₋₆ alkyl) amino group, more preferably a C₁₋₃ alkyl group which is substituted with a di(C₁₋₃ alkyl) amino group, and still more preferably a dimethylaminomethyl group.

The C₁₋₆ alkyl group of the C₁₋₆ alkyl group which may be substituted, as R², is preferably a C₁₋₃ alkyl group and more preferably a methyl group.

The substituent of each of the C₁₋₆ alkyl group which may be substituted, the C₂₋₆ alkenyl group which may be substituted, or the C₂₋₆ alkynyl group which may be substituted, as R², is preferably a di(C₁₋₆ alkyl) amino group which may be substituted with one or more groups selected from the substituent group A-1 or a heterocyclic group which may be substituted with one or more groups selected from the substituent group A-1, and more preferably a di(C₁₋₆ alkyl) amino group which may be substituted with one or more groups selected from the substituent group A-1.

The di(C₁₋₆ alkyl) amino group of the di(C₁₋₆ alkyl) amino group which may be substituted with one or more groups selected from the substituent group A-1 is preferably a di(C₁₋₃ alkyl) amino group and more preferably a dimethylamino group.

The heterocyclic group of the heterocyclic group which may be substituted with one or more groups selected from the substituent group A-1 is preferably an azetidinyl group, a piperazinyl group, or a morpholinyl group.

The substituent group A-1: a halogen atom, a hydroxyl group which may be protected, and a C₁₋₆ alkyl group which may be substituted with a hydroxyl group.

R³ is a hydrogen atom, a C₁₋₆ alkyl group which may be substituted, a C₂₋₆ alkenyl group which may be substituted, or a C₂₋₆ alkynyl group which may be substituted, preferably a hydrogen atom or a C₁₋₆ alkyl group, and more preferably a C₁₋₆ alkyl group.

In any case where other substituents are any substituents, the C₁₋₆ alkyl group, the C₂₋₆ alkenyl group, or the C₂₋₆ alkynyl group, as R³, may be substituted with one or more groups selected from a halogen atom, a cyano group, an amino group which may be protected, a hydroxyl group which may be protected, an aryl group which may be substituted with one or more groups selected from the substituent group A, and a heterocyclic group which may be substituted with one or more groups selected from the substituent group A.

The C₁₋₆ alkyl group of the C₁₋₆ alkyl group which may be substituted, as R³, is preferably a C₁₋₃ alkyl group and more preferably a methyl group.

m is an integer of 1 to 3, preferably an integer of 1 or 2, and more preferably an integer of 1.

m pieces of R⁴'s are the same or different from each other, are a hydrogen atom or a C₁₋₆ alkyl group which may be substituted, and are preferably a hydrogen atom.

In any case where other substituents are any substituents, the C₁₋₆ alkyl group as R⁴ may be substituted with one or more groups selected from a halogen atom, a cyano group, an amino group which may be protected, and a hydroxyl group which may be protected.

One R⁴ selected from the m pieces of R⁴'s may be combined together with R³ to form a C₁₋₆ alkylene group which may be substituted, and the C₁₋₆ alkylene group of the C₁₋₆ alkylene group which may be substituted is preferably a C₁₋₃ alkylene group and more preferably a propylene group. The substituent of the C₁₋₆ alkylene group which may be substituted is preferably a halogen atom, a hydroxyl group, or a C₁₋₃ alkoxy group, more preferably a fluorine atom, a hydroxyl group, or a methoxy group, and still more preferably a fluorine atom or a methoxy group.

m pieces of R⁵'s are the same or different from each other, are a hydrogen atom, a C₁₋₆ alkyl group which may be substituted, a C₂₋₆ alkenyl group which may be substituted, or a C₂₋₆ alkynyl group which may be substituted, and preferably a C₁₋₆ alkyl group which may be substituted.

In any case where other substituents are any substituents, the C₁₋₆ alkyl group, the C₂₋₆ alkenyl group which may be substituted, or the C₂₋₆ alkynyl group which may be substituted, as R⁵, may be substituted with one or more groups selected from a halogen atom, a cyano group, an amino group which may be protected, and a hydroxyl group which may be protected.

The C₁₋₆ alkyl group of the C₁₋₆ alkyl group which may be substituted, as R⁵, is preferably a C₁₋₃ alkyl group and more preferably a methyl group.

n is an integer of 0 to 3, preferably an integer of 0 or 1, and more preferably an integer of 0.

n pieces of R⁶'s are the same or different from each other and are a hydrogen atom or a C₁₋₆ alkyl group which may be substituted, preferably a hydrogen atom or a C₁₋₆ alkyl group, and ore preferably a hydrogen atom.

n pieces of R⁷'s are the same or different from each other and are a hydrogen atom or a C₁₋₆ alkyl group which may be substituted, preferably a hydrogen atom or a C₁₋₆ alkyl group, and more preferably a hydrogen atom.

In any case where other substituents are any substituents, the C₁₋₆ alkyl group as R⁶ and R⁷ may be substituted with a halogen atom, a cyano group, an amino group which may be protected, or a hydroxyl group which may be protected.

R⁸ is a hydrogen atom, a C₁₋₆ alkyl group which may be substituted, a C₂₋₆ alkenyl group which may be substituted, or a C₂₋₆ alkynyl group which may be substituted, and preferably a hydrogen atom.

In any case where other substituents are any substituents, the C₁₋₆ alkyl group, the C₂₋₆ alkenyl group, or the C₂₋₆ alkynyl group, as R⁸, may be substituted with one or more groups selected from a halogen atom, a cyano group, an amino group which may be protected, and a hydroxyl group which may be protected.

R⁹ is a C₁₋₆ alkyl group which may be substituted, a C₂₋₆ alkenyl group which may be substituted, a C₂₋₆ alkynyl group which may be substituted, or a C₃₋₈ cycloalkyl group which may be substituted, preferably a C₁₋₆ alkyl group which may be substituted or a C₃₋₈ cycloalkyl group which may be substituted, and more preferably a C₁₋₆ alkyl group which may be substituted.

In any case where other substituents are any substituents, the C₁₋₆ alkyl group, the C₂₋₆ alkenyl group, the C₂₋₆ alkynyl group, or the C₃₋₈ cycloalkyl group, as R⁹, may be substituted with one or more groups selected from a halogen atom, a cyano group, an amino group which may be protected, a hydroxyl group which may be protected, and a C₁₋₆ alkoxy group which may be substituted with one or more groups selected from the substituent group A.

The C₁₋₆ alkyl group which may be substituted, as R⁹, is preferably a C₁₋₆ alkyl group which may be substituted.

The C₁₋₆ alkyl group of the C₁₋₆ alkyl group which may be substituted, as R⁹, is preferably a C₁₋₃ alkyl group.

The substituent of the C₁₋₆ alkyl group as R⁹, which may be substituted, is preferably a halogen atom or a C₁₋₃ alkoxy group and more preferably a methoxy group.

R⁸ and R⁹ may be combined together with a nitrogen atom to which R⁸ and R⁹ are bonded, to form a cyclic amino group which may be substituted, where the cyclic amino group which may be substituted is preferably a morpholinyl group.

In any case where other substituents are any substituents, the cyclic amino group formed by combining R⁸ and R⁹ together with the nitrogen atom to which R⁸ and R⁹ are bonded may be substituted with one or more groups selected from a halogen atom, a cyano group, an amino group which may be protected, a hydroxyl group which may be protected, and an oxo group.

R¹⁰ is a hydrogen atom, a C₁₋₆ alkyl group which may be substituted, a C₂₋₆ alkenyl group which may be substituted, or a C₂₋₆ alkynyl group which may be substituted, and preferably a hydrogen atom.

In any case where other substituents are any substituents, the C₁₋₆ alkyl group, the C₂₋₆ alkenyl group, or the C₂₋₆ alkynyl group, as R¹⁰, may be substituted with one or more groups selected from a halogen atom, a cyano group, an amino group which may be protected, a hydroxyl group which may be protected, and a C₁₋₆ alkoxy group which may be substituted with one or more groups selected from the substituent group A.

R¹¹ is a C₁₋₆ alkyl group which may be substituted, a C₂₋₆ alkenyl group which may be substituted, a C₂₋₆ alkynyl group which may be substituted, a C₃₋₈ cycloalkyl group which may be substituted, an aryl group which may be substituted, or a heterocyclic group which may be substituted, preferably a C₁₋₆ alkyl group which may be substituted, an aryl group which may be substituted, or a heterocyclic group which may be substituted, more preferably an aryl group which may be substituted or a heterocyclic group which may be substituted, and still more preferably an aryl group which may be substituted.

In any case where other substituents are any substituents, the C₁₋₆ alkyl group, the C₂₋₆ alkenyl group, the C₂₋₆ alkynyl group, the C₃₋₈ cycloalkyl group, the aryl group, or the heterocyclic group, as R¹¹, may be substituted with one or more groups selected from a halogen atom, a cyano group, an amino group which may be protected, a hydroxyl group which may be protected, and a C₁₋₆ alkoxy group which may be substituted with one or more groups selected from the substituent group A.

The substituent of each, as R¹¹, of the C₁₋₆ alkyl group which may be substituted, the C₃₋₈ cycloalkyl group which may be substituted, the aryl group which may be substituted, or the heterocyclic group which may be substituted, is preferably a C₁₋₆ alkoxy group which may be substituted with one or more groups selected from the substituent group A-2.

The substituent group A-2: a halogen atom, a C₁₋₆ alkyl group, a C₃₋₈ cycloalkyl group, a C₁₋₆ alkoxy group, and a heterocyclic group.

The C₁₋₆ alkyl group which may be substituted, as R¹¹, is preferably a C₁₋₆ alkyl group which is substituted, more preferably a C₁₋₃ alkyl group which is substituted, and still more preferably an ethyl group which is substituted.

In a case where R¹¹ is a C₁₋₆ alkyl group which is substituted, the substituent of the C₁₋₆ alkyl group is preferably a heterocyclic group, more preferably a pyridyl group, a pyrrolidinyl group, or a morpholinyl group.

The aryl group which may be substituted, as R¹¹, is preferably an aryl group which is substituted, more preferably a phenyl group which is substituted.

In a case where R¹¹ is a phenyl group which is substituted, the substituent of the phenyl group is preferably a halogen atom, a cyano group, or a carbamoyl group and more preferably a fluorine atom or a cyano group.

In a case where R¹¹ is a phenyl group which is substituted, the phenyl group preferably has no substituent at the o-position but has a substituent at the m-position or the p-position, and more preferably has a substituent only at the p-position.

The preferred substituent at the m-position or p-position is as described above.

The heterocyclic group which may be substituted, as R¹¹, is preferably a pyridyl group which may be substituted, an indazolyl group which may be substituted, a pyrazolopyridinyl group which may be substituted, or an isoquinolyl group which may be substituted.

X¹ is an oxygen atom, N(R²⁰) (in the formula, R²⁰ has the same meaning as the above), C(=O), C(=O)-N(R²⁰) (in the formula, R²⁰ has the same meaning as the above), or a bond and is preferably C(=O)-N(R²⁰) (in the formula, R²⁰ has the same meaning as the above).

R²⁰ is a hydrogen atom, a C₁₋₆ alkyl group which may be substituted, a C₂₋₆ alkenyl group which may be substituted, or a C₂₋₆ alkynyl group which may be substituted and is preferably a hydrogen atom.

In any case where other substituents are any substituents, the C₁₋₆ alkyl group, the C₂₋₆ alkenyl group, or the C₂₋₆ alkynyl group, as R²⁰, may be substituted with one or more groups selected from a halogen atom, a cyano group, an amino group which may be protected, and a hydroxyl group which may be protected.

X² is a C₁₋₆ alkylene group which may be substituted, a divalent alicyclic hydrocarbon group which may be substituted, or a divalent aromatic hydrocarbon group which may be substituted and preferably a C₁₋₆ alkylene group which may be substituted or a divalent alicyclic hydrocarbon group which may be substituted.

In any case where other substituents are any substituents, the C₁₋₆ alkylene group, the divalent alicyclic hydrocarbon group, or the divalent aromatic hydrocarbon group, as X², may be substituted with one or more groups selected from a halogen atom, a cyano group, an amino group which may be protected, and a hydroxyl group which may be protected.

The C₁₋₆ alkylene group which may be substituted, as X², is preferably a C₁₋₆ alkylene group which is unsubstituted.

The C₁₋₆ alkylene group of the C₁₋₆ alkylene group which may be substituted, as X², is preferably a methylene group, an ethylene group, or a trimethylene and more preferably a trimethylene group.

The substituent of the C₁₋₆ alkylene group which may be substituted, as X², is preferably a C₁₋₆ alkyl group, more preferably a C₁₋₃ alkyl group, and still more preferably an ethyl group.

The divalent alicyclic hydrocarbon group which may be substituted, as X², is preferably a divalent alicyclic hydrocarbon group which is unsubstituted.

The divalent alicyclic hydrocarbon group of the divalent alicyclic hydrocarbon group which may be substituted, as X², is preferably cyclobutylene group or cyclohexylene group and more preferably a cyclobutylene group.

In a case of being a cyclobutylene group, X² is preferably a cyclobutylene group represented by Formula [2] 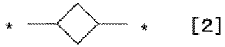 (in the formula, ^{∗} indicates a bonding position) and more preferably a cyclobutylene group represented by Formula [3] 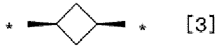 (in the formula, ^{∗} indicates a bonding position).

In a case of being a cyclohexylene group, X² is preferably a cyclohexylene group represented by Formula [4] 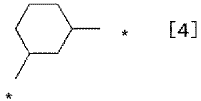 (in the formula, ^{∗} indicates a bonding position).

The divalent aromatic hydrocarbon group of the divalent aromatic hydrocarbon group which may be substituted, as X², is preferably a phenylene group.

In a case of being a phenylene group, X² is preferably a phenylene group represented by Formula [5] 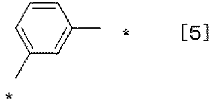 (in the formula, ^{∗} indicates a bonding position).

The substituent of the divalent aromatic hydrocarbon group as X², which may be substituted, is preferably a halogen atom or a C₁₋₆ alkyl group.

In a case of being a halogen atom, the substituent is preferably a chlorine atom.

In a case of being a C₁₋₆ alkyl group, the substituent is preferably a C₁₋₃ alkyl group and more preferably a methyl group.

X³ is a C₁₋₆ alkylene group which may be substituted, a C₂₋₆ alkenylene group which may be substituted, a C₂₋₆ alkynylene group which may be substituted, or N(R²⁰)-C(=O) (in the formula, R²⁰ has the same meaning as the above), preferably a C₂₋₆ alkynylene group which may be substituted or N(R²⁰)-C(=O) (in the formula, R²⁰ has the same meaning as the above), and more preferably a C₂₋₆ alkynylene group which may be substituted.

In any case where other substituents are any substituents, the C₁₋₆ alkylene group, the C₂₋₆ alkenylene group, or the C₂₋₆ alkynylene group, as X³, may be substituted with one or more groups selected from a halogen atom, a cyano group, an amino group which may be protected, and a hydroxyl group which may be protected.

The C₂₋₆ alkynylene group of the C₂₋₆ alkynylene group which may be substituted, as X³, is preferably an ethynylene group.

Examples of the salt of the compound of General Formula [1] include a salt of a generally known basic group such as an amino group and a salt of a generally known acidic group such as a hydroxyl group or a carboxyl group.

Examples of the salt of the basic group include a salt with a mineral acid such as hydrochloric acid, hydrobromic acid, nitric acid, or sulfuric acid; a salt with an organic carboxylic acid such as formic acid, acetic acid, citric acid, oxalic acid, fumaric acid, maleic acid, succinic acid, malic acid, tartaric acid, aspartic acid, trichloroacetic acid, or trifluoroacetic acid; and a salt with a sulfonic acid such as methanesulfonic acid, benzenesulfonic acid, p-toluenesulfonic acid, mesitylenesulfonic acid, or naphthalenesulfonic acid.

Among the salts described above, examples of the preferred salt include a pharmacologically acceptable salt. A more preferred salt is a succinate salt.

The salt may be an anhydride, a hydrate, or a solvate.

Specific examples of Compound A (the compound represented by General Formula [1]) include the compounds described in Tables 1-1 to 1-4 after paragraph 0130 of WO2016/027904A.

The particularly preferred compound is (S,E)-N-(1-((5-(2-((4-cyanophenyl)amino)-4-(propylamino)pyrimidin-5-yl)-4-pentyn-1-yl)ami no)-1-oxopropan-2-yl)-4-(dimethylamino)-N-methyl-2-butenamide (Compound 38 of WO2016/027904A) and this compound is particularly referred to as Compound A1 in the present specification.

Compound A1 may also be referred to as (S,E)-N-{1-[(5-{2-[(4-cyanophenyl)amino]-4-(propylamino)pyrimidin-5-yl}pent-4-yn-1-yl)a mino]-1-oxopropan-2-yl}-4-(dimethylamino)-N-methylbut-2-enamide.

Compound A may be a compound or a salt thereof, which is represented by General Formula [1] of WO2013/157540A, and the description thereof can be referred to and taken into consideration, the contents of which are incorporated in the present specification.

In addition, other preferred compounds of Compound A include the followings.
(S,E)-4-(dimethylamino)-N-(1-((5-(2-((3-fluorophenyl)amino)-4-(propylamino)pyrim idin-5-yl)pent-4-yn-1-yl)amino)-1-oxopropan-2-yl)-N-methylbut-2-enamide (Compound 34 of WO2016/027904A),
(E)-4-(dimethylamino)-N-((S)-1-(((1s,3R)-3-((2-((3-fluorophenyl)amino)-4-(propyla mino)pyrimidin-5-yl)ethynyl)cyclobutyl)amino)-1-oxopropan-2-yl)-N-methylbut-2-enamide (Compound 39 of WO2016/027904A),
(E)-N-((S)-1-(((1s,3R)-3-((4-(cyclopropylamino)-2-((4-fluorophenyl)amino)pyrimidi n-5-yl)ethynyl)cyclobutyl)amino)-1-oxopropan-2-yl)-4-(dimethylamino)-N-methylbut-2-enam ide (Compound 40 of WO2016/027904A), and
(E)-4-(dimethylamino)-N-((S)-1-(((1s,3R)-3-((2-((4-fluorophenyl)amino)-4-(methyla mino)pyrimidin-5-yl)ethynyl)cyclobutyl)amino)-1-oxopropan-2-yl)-N-methylbut-2-enamide (Compound 41 of WO2016/027904A).

Structures of the suitable compounds are shown below.

| | Structure | | | Structure |
|---|---|---|---|---|
| 34 | 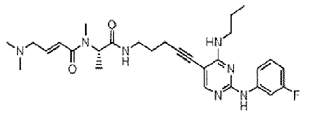 | | 40 | 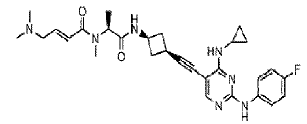 |
| 38 | 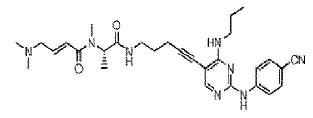 | | 41 | 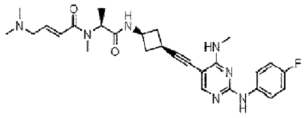 |
| 39 | 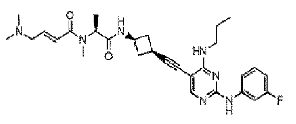 | | | |

Next, a method for producing Compound A will be described. Compound A can be produced, for example, by the method disclosed in WO2017/010535A. Further, a salt of Compound A can be produced by the method disclosed in WO2015/056683A.

### <BCL-2 inhibitor and pyrimidine antimetabolite>

A BCL-2 inhibitor is a drug that targets BCL-2 (B-cell lymphoma-2) which is an anti-apoptotic protein.

Examples of the BCL-2 inhibitor include venetoclax, 4-[4-[[2-(4-chlorophenyl)-5,5-dimethyl-1-cyclohexen-1-yl] methyl]-1-piperazinyl] -N- [[4-[[(1R )-3-(4-morpholinyl)-1-[(phenylthio)methyl]propyl]amino]-3-[(trifluoromethyl) sulfonyl]phenyl ] sulfonyl]benzamide (also known as ABT-263, disclosed in PCT Publication WO2009/155386A); tetrocarcin A; antimycin; gossypol ((-) BL-193); obatoclax; ethyl-2-amino-6-cyclopentyl-4-(1-cyano-2-ethoxy-2-oxoethyl)-4H-chromon-3-carboxylate (HA14-1); oblimersen (G3139, Genasense (registered trade mark)); a Bak BH3 peptide; (-)-gossypol acetic acid (AT-101); 4-[4-[(4'-chloro[1,1'-biphenyl]-2-yl)methyl]-1-piperazinyl]-N-[[4-[[(1R)-3-(dimethylamino)-1-[(phenylthio)methyl]propyl] amino]-3-nitrophenyl] sulfonyl] -benzamide (ABT-737, CAS 852808-04-9); and navitoclax (ABT-263, CAS 923564-51-6). However, the BCL-2 inhibitor is not particularly limited. The BCL-2 inhibitor is preferably venetoclax or a salt thereof.

Venetoclax is a selective BCL-2 inhibitor that selectively binds, with a strong affinity, to the anti-apoptotic protein BCL-2 which is involved in many types of hematological cancer. The BCL-2 protein contributes to the survival of leukemia cells by binding to a apoptosis-promoting protein, whereas it is presumed that venetoclax promotes apoptosis of leukemia cells by binding to BCL-2, thereby releasing the apoptosis-promoting protein. The structure of venetoclax is shown below.

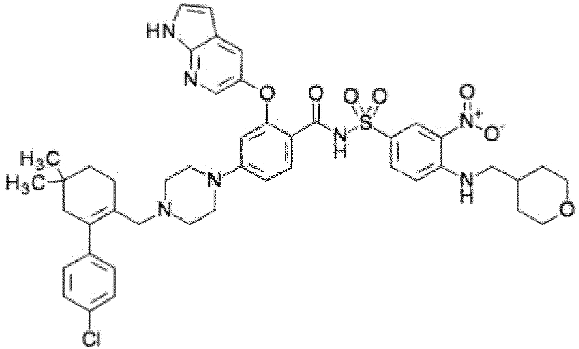

Pyrimidine antimetabolites have structures similar to substrates or enzymes required for DNA replication in cells, and after being incorporated into cells, they become active substances by the action of the respective enzymes, and inhibits DNA synthesis or RNA synthesis or inhibits DNA methylation.

As the pyrimidine antimetabolite, a compound selected from the group consisting of azacytidine, decitabine, guadecitabine, cytarabine, and gemcitabine, or a salt or hydrate thereof can be preferably used; however, it is not particularly limited. Examples of the salt of the above compound include those described above as the salt of the compound of Formula [1], and a pharmacologically acceptable salt is preferable.

### <Combination medicine>

In the combination medicine of according to the embodiment of the invention, Compound A and at least one selected from the group consisting of a BCL-2 inhibitor and a pyrimidine antimetabolite may be provided as the same composition or as the separate compositions.

### The daily dose of Compound A is preferably 10 to 450 mg.

In a case where Compound A is administered twice a day (a BID administration), the dose per administration is preferably 5 to 225 mg. In the BID administration, the dose per administration is preferably 10 to 150 mg and more preferably 15 to 100 mg. In the BID administration, the lower limit value of the dose per administration is 5 mg, preferably 10 mg, more preferably 15 mg, and particularly preferably 20 mg. In the BID administration, the upper limit value of the dose per administration is 225 mg, preferably 200 mg, more preferably 150 mg, and particularly preferably 130 mg.

In a case where Compound A is administered thrice a day (a TID administration), the dose per administration is preferably 5 to 150 mg. In the TID administration, the dose per administration is preferably 10 to 150 mg and more preferably 15 to 100 mg. In the TID administration, the lower limit value of the dose per administration is 5 mg, preferably 10 mg, more preferably 15 mg, and particularly preferably 20 mg.

Compound A is preferably administered orally.

Examples of the formulation form of Compound A include an oral agent, and examples the oral agent include a capsule agent. The administration formulation form can be produced by a conventional formulation producing method known to those skilled in the art.

In a case where a pyrimidine antimetabolite is used, the daily dose of the pyrimidine antimetabolite is preferably 1 to 3,000 mg/m².

In a case where the pyrimidine antimetabolite is decitabine or a salt or hydrate thereof, the daily dose of decitabine or the salt or hydrate thereof is preferably 1 to 80 mg/m².

The pyrimidine antimetabolite is preferably administered intravenously drip infusion.

Examples of the formulation form of the pyrimidine antimetabolites include a drip infusion preparation, and examples of the drip infusion preparation include a liquid preparation. The administration formulation form can be produced by a conventional formulation producing method known to those skilled in the art.

In a case where a BCL-2 inhibitor is used, Compound A and the BCL-2 inhibitor can be provided as the same composition.

In a case where a BCL-2 inhibitor is used, the daily dose of the BCL-2 inhibitor is preferably 20 mg to 600 mg.

It is preferable to contain 0.3% to 50% by mass of the compound represented by General Formula [1] or a salt thereof and preferable to 40% to 99.7% by mass (preferably 50% to 99.7% by mass and more preferably 60% to 99.7% by mass) of a BCL-2 inhibitor with respect to the total amount of the combination medicine. However, the total of both does not exceed 100% by mass.

Examples of the same composition containing Compound A and the BCL-2 inhibitor include an oral preparation, and examples of the oral agent include a tablet. The administration formulation form of the tablet can be produced by a conventional formulation producing method known to those skilled in the art.

The combination medicine according to the embodiment of the invention can be effectively used for the treatment of tumors, particularly hematological cancer (for example, acute myeloid leukemia). The combination medicine according to the embodiment of the invention can be used as an anti-cancer agent.

The present invention provides a method for using Compound A and at least one selected from the group consisting of a BCL-2 inhibitor and a pyrimidine antimetabolite to treat a tumor, the method including administering Compound A and the at least one selected from the group consisting of a BCL-2 inhibitor and a pyrimidine antimetabolite to a subject (a mammal, including a human) in need of tumor treatment.

The present invention provides a method for treating a tumor, including administering Compound A and at least one selected from the group consisting of a BCL-2 inhibitor and a pyrimidine antimetabolite to a subject (a mammal, including a human) in need of tumor treatment.

The present invention provides the use of a combination of Compound A and at least one selected from the group consisting of a BCL-2 inhibitor and a pyrimidine antimetabolite, for producing an anti-tumor agent.

The present invention provides a combination of Compound A and at least one selected from the group consisting of a BCL-2 inhibitor and a pyrimidine antimetabolite, for use in the curing of a tumor.

### Examples

The present invention will be described in more detail below with reference to Examples; however, the present invention is not limited to these Examples.

### <Preparation of succinate salt of Compound A1>

(S,E)-N-(1-((5-(2-((4-cyanophenyl)amino)-4-(propylamino)pyrimidin-5-yl)-4-pentyn-1-yl)amino)-1-oxopropan-2-yl)-4-(dimethylamino)-N-methyl-2-butenamide was synthesized according to the method disclosed in Examples of WO2017/010535A, converted to a succinate salt thereof according to the method described in Examples in WO2015/056683A, and used in the following tests.

### <Evaluation of proliferation inhibitory activity against FLT3-ITD mutation-positive leukemia cell line MV-4-11 by combined use of anti-cancer agent>

Decitabine (hereinafter, also referred to as DEC), venetoclax (hereinafter, also referred to as VEN), and a succinate salt of Compound A1 were used as test substances.

Decitabine (Cat. # A2232, manufactured by Tokyo Chemical Industry Co., Ltd.), venetoclax (Cat. # V-3579, manufactured by LC Laboratories), and the succinate salt of Compound A1 were dissolved in dimethyl sulfoxide (DMSO) and used.

MV-4-11 cells which are a human FLT3-ITD mutation-positive leukemia cell line were subcultured in an RPMI-1640 medium containing 10% serum. During this test, all cell cultures were performed in a CO₂ incubator (setting: 37°C, 5% CO₂, water vapor saturated). Cells were diluted with a medium containing 10% serum to 1,000 cells/well/20 µL and seeded on a 384-well plate.

Decitabine, venetoclax, and the succinate salt of Compound A1 were each dissolved in DMSO to prepare a 20 mmol/L DMSO solution. After being serially diluted with DMSO and further being diluted with a medium containing 10% serum, each of the test substance solutions having a concentration 10 times the final treatment concentration was prepared. The maximum concentration of the Compound A1 solution was set to 40 nmol/L or 20 nmol/L, the maximum concentration of the decitabine solution was set to 60 µmol/L, and the maximum concentration of the venetoclax solution was set to 1.2 µmol/L, and then nine concentrations were combined at a common ratio of 1/2 and used.

2.5 µL of Compound A1 solution was added to each well, and further, 2.5 µL of the decitabine solution or the venetoclax solution was added to each well. In addition, a group (a positive control group) in which only a solvent containing no drugs was added to a well in which cells were plated and a group (a negative control group) in which only a solvent containing no drugs was added to a well in which only a medium was added were prepared.

After the addition of the drug, cells were cultured for 3 days, and the cell viability was evaluated using CellTiter-Glo (registered trade mark) Reagent (Cat. # G7570, manufactured by Promega Corporation) using the amount of ATP in the cells as an indicator.

The suppression rate was determined by assuming that the amount of luminescence signal in the negative control group corresponded to the suppression of cell viability by 100% and the amount of luminescence signal in the positive control group corresponded to the suppression of cell viability by 0%. The concentration (IC50 value) at which cell viability is suppressed by 50% was calculated using XLFit (registered trade mark) software Ver. 3 (manufactured by ITOCHU Techno-Solutions Corporation). The suppression effect (Fa, the fraction affected by the dose) was calculated by dividing the suppression rate by 100.

The combination effect of the present invention was determined by calculating the combination index (CI), which is a quantitative indicator of the combination effect, using CalcuSyn (BIOSOFT) based on the value of the suppression effect.

Table 1 shows the CI results (the average of 4 experiments) when Compound A1 and decitabine were used in combination (concentration fixing ratio, 1:3,000) and Compound A1 and venetoclax were used in combination (concentration fixing ratio, 1:120). In the table, "Fa around 0.5" and "Fa around 0.75" mean the effect of suppressing the cell viability by about 50% and about 75%, respectively, in a case where drugs are used in combination.

**[Table 1]**

| | Fa around 0.5 | Fa around 0.75 |
|---|---|---|
| Decitabine | + + + | + + + |
| Venetoclax | + + | + + + |

In the table, the determination criteria for CI are as follows according to the CalcuSyn manual.
+++: Less than 0.7, a strong synergistic effect
++: 0.7 or more and less than 0.85, a moderate synergistic effect
+: 0.85 or more and less than 0.90, a weak synergistic effect
±: 0.90 or more and less than 1.10, an additive effect
-: 1.10 or more and less than 1.20, a weak antagonistic effect
--: 1.20 or more and less than 1.45, a moderate antagonistic effect
---: 1.45 or more, a strong antagonistic effect.

In a case where Compound A1 and decitabine were used in combination, a strong synergistic effect was observed in the suppression effects around 0.5 and 0.75.

In a case where Compound A1 and venetoclax were used in combination, a moderate synergistic effect was observed in the suppression effect around 0.5, and a strong synergistic effect was observed around 0.75.

### <Evaluation of proliferation inhibitory activity against cell derived from leukemia patient by combined use of existing anti-cancer agent>

Decitabine, venetoclax, and Compound A1 were used as test substances, and each of those dissolved in DMSO was used for this evaluation.

Monocytes isolated from bone marrow or peripheral blood of two leukemia patients (one patient is FLT3-ITD mutation-positive, and the other patient is FLT3-ITD mutation-negative) were used and cultured in MethoCult^{™} H4534 Classic Without EPO (Cat. # H4534, manufacture by STEMCELL technologies). During this test, all cell cultures were performed in a CO₂ incubator (setting: 37°C, 5% CO₂, water vapor saturated). Cells were diluted with medium to 5,000 cells/well/90 µL and seeded on a 96-well plate.

Decitabine, venetoclax, and Compound A1 were each dissolved in DMSO to prepare a 20 mmol/L DMSO solution. After serially diluting with DMSO and further diluting with a medium, each of the test substance solutions having a concentration 10 times the final treatment concentration was prepared. The maximum concentration of the Compound A1 solution was set to 800 nmol/L, the maximum concentration of the decitabine solution was set to 8 µmol/L, and the maximum concentration of the venetoclax solution was set to 800 nmol/L, and then six concentrations were combined at a common ratio of 1/5 and used.

10 µL of a solution obtained by mixing equal amounts of the Compound A1 solution, and the decitabine solution or the venetoclax solution, was added to each well. In addition, a group (a positive control group) in which only a solvent containing no drugs was added to a well in which cells were plated and a group (a negative control group) in which only a solvent containing no drugs was added to a well in which only a medium was added were prepared.

After the addition of the drug, cells were cultured for 7 or 8 days, and the cell viability was evaluated using CellTiter-Glo (registered trade mark) Reagent (Cat. # G7570, manufactured by Promega Corporation) using the amount of ATP in the cells as an indicator.

The suppression rate was determined by assuming that the amount of luminescence signal in the negative control group corresponded to the suppression of cell viability by 100% and the amount of luminescence signal in the positive control group corresponded to the suppression of cell viability by 0%. The concentration (IC50 value) at which cell viability is suppressed by 50% was calculated using XLFit (registered trade mark) software Ver. 3 (manufactured by ITOCHU Techno-Solutions Corporation). The suppression effect (Fa, the fraction affected by the dose) was calculated by dividing the suppression rate by 100.

The combination effect of the present invention was determined by calculating the combination index (CI), which is a quantitative indicator of the combination effect, using CalcuSyn (BIOSOFT) based on the value of the suppression effect.

Table 2 shows the CI results when Compound A1 and decitabine were used in combination (concentration fixing ratio, 1:10) and Compound A1 and venetoclax were used in combination (concentration fixing ratio, 1:1). In the table, "Fa around 0.5" and "Fa around 0.75" mean the effect of suppressing the cell viability by about 50% and about 75%, respectively, in a case where drugs are used in combination.

**[Table 2]**

| Cell derived from FLT3-ITD mutation-positive leukemia patient | | |
|---|---|---|
| | Fa around 0.5 | Fa around 0.75 |
| Decitabine | + + + | + + + |
| Venetoclax | + + + | + + + |

| Cell derived from FLT3-ITD mutation-negative leukemia patient | | |
|---|---|---|
| | Fa around 0.5 | Fa around 0.75 |
| Decitabine | + + + | + + + |
| Venetoclax | + + + | + + + |

In the table, the determination criteria for CI are as follows according to the CalcuSyn manual.
+++: Less than 0.7, a strong synergistic effect
++: 0.7 or more and less than 0.85, a moderate synergistic effect
+: 0.85 or more and less than 0.90, a weak synergistic effect
±: 0.90 or more and less than 1.10, an additive effect
-: 1.10 or more and less than 1.20, a weak antagonistic effect
--: 1.20 or more and less than 1.45, a moderate antagonistic effect
---: 1.45 or more, a strong antagonistic effect.

In cells derived from FLT3-ITD mutation-positive and FLT3-ITD mutation-negative leukemia patients, in a case where Compound A1 and decitabine were used in combination, a strong synergistic effect was observed in the suppression effects around 0.5 and 0.75.

In cells derived from the same mutation-positive and the same mutation-negative leukemia patients, in a case where Compound A1 and venetoclax were used in combination, a strong synergistic effect was observed in the suppression effects around 0.5 and 0.75.

The combination medicine according to the embodiment of the present invention exhibits a curing effect on a tumor such as acute myeloid leukemia and thus useful.

## Claims

1. A combination medicine comprising:
a compound represented by General Formula [1] or a salt thereof; and
at least one selected from the group consisting of a BCL-2 inhibitor and a pyrimidine antimetabolite, 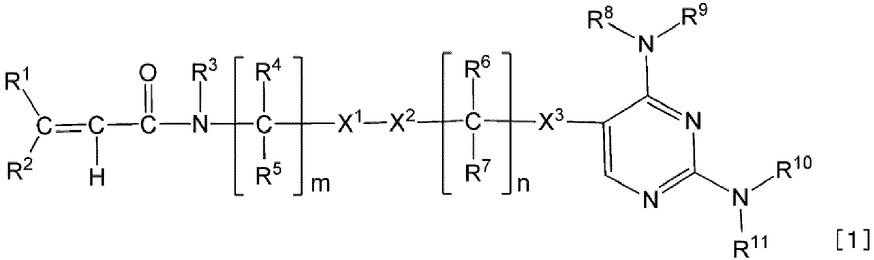
in the formula,
R¹ represents a hydrogen atom or a C₁₋₆ alkyl group which may be substituted,
R² represents a hydrogen atom, a C₁₋₆ alkyl group which may be substituted, a C₂₋₆ alkenyl group which may be substituted, or a C₂₋₆ alkynyl group which may be substituted,
R³ represents a hydrogen atom, a C₁₋₆ alkyl group which may be substituted, a C₂₋₆ alkenyl group which may be substituted, or a C₂₋₆ alkynyl group which may be substituted,
m represents an integer of 1 to 3,
m pieces of R⁴'s may be the same or different from each other and represent a hydrogen atom or a C₁₋₆ alkyl group which may be substituted, where one R⁴ selected from the m pieces of R⁴'s may be combined together with R³ to form a C₁₋₆ alkylene group which may be substituted,
m pieces of R⁵'s may be the same or different from each other and represent a hydrogen atom, a C₁₋₆ alkyl group which may be substituted, a C₂₋₆ alkenyl group which may be substituted, or a C₂₋₆ alkynyl group which may be substituted,
X¹ represents an oxygen atom, N(R²⁰) (in the formula, R²⁰ represents a hydrogen atom, a C₁₋₆ alkyl group which may be substituted, a C₂₋₆ alkenyl group which may be substituted, or a C₂₋₆ alkynyl group which may be substituted), C(=O), C(=O)-N(R²⁰) (in the formula, R²⁰ has the same meaning as above), or a bond,
X² represents a C₁₋₆ alkylene group which may be substituted, a divalent alicyclic hydrocarbon group which may be substituted, or a divalent aromatic hydrocarbon group which may be substituted,
n represents an integer of 0 to 3,
n pieces of R⁶'s may be the same or different from each other and represent a hydrogen atom or a C₁₋₆ alkyl group which may be substituted,
n pieces of R⁷'s may be the same or different from each other and represent a hydrogen atom or a C₁₋₆ alkyl group which may be substituted,
X³ represents a C₁₋₆ alkylene group which may be substituted, a C₂₋₆ alkenylene group which may be substituted, a C₂₋₆ alkynylene group which may be substituted, or N(R²⁰)-C(=O) (in the formula, R²⁰ has the same meaning as the above),
R⁸ represents a hydrogen atom, a C₁₋₆ alkyl group which may be substituted, a C₂₋₆ alkenyl group which may be substituted, or a C₂₋₆ alkynyl group which may be substituted,
R⁹ represents a C₁₋₆ alkyl group which may be substituted, a C₂₋₆ alkenyl group which may be substituted, a C₂₋₆ alkynyl group which may be substituted, or a C₃₋₈ cycloalkyl group which may be substituted,
R⁸ and R⁹ may be combined together with a nitrogen atom to which R⁸ and R⁹ are bonded, to form a cyclic amino group which may be substituted,
R¹⁰ represents a hydrogen atom, a C₁₋₆ alkyl group which may be substituted, a C₂₋₆ alkenyl group which may be substituted, or a C₂₋₆ alkynyl group which may be substituted, and
R¹¹ represents a C₁₋₆ alkyl group which may be substituted, a C₂₋₆ alkenyl group which may be substituted, a C₂₋₆ alkynyl group which may be substituted, a C₃₋₈ cycloalkyl group which may be substituted, an aryl group which may be substituted, or a heterocyclic group which may be substituted.

2. The combination medicine according to claim 1,
wherein R¹⁰ is a hydrogen atom, and
X¹ is C(=O)-N(R²⁰) (in the formula, R²⁰ represents a hydrogen atom, a C₁₋₆ alkyl group which may be substituted, a C₂₋₆ alkenyl group which may be substituted, or a C₂₋₆ alkynyl group which may be substituted).

3. The combination medicine according to claim 1 or 2,
wherein X³ is a C₂₋₆ alkynylene group which may be substituted.

4. The combination medicine according to any one of claims 1 to 3,
wherein the compound represented by General Formula [1] is (S,E)-N-(1-((5-(2-((4-cyanophenyl)amino)-4-(propylamino)pyrimidin-5-yl)-4-pentyn-1-yl)ami no)-1-oxopropan-2-yl)-4-(dimethylamino)-N-methyl-2-butenamide.

5. The combination medicine according to any one of claims 1 to 4,
wherein a daily dose of the compound represented by General Formula [1] or the salt thereof is 10 to 450 mg.

6. The combination medicine according to any one of claims 1 to 5,
wherein the compound represented by General Formula [1] or the salt thereof and at least one selected from the group consisting of venetoclax or a salt thereof and a pyrimidine antimetabolite are provided as the same composition or as separate compositions.

7. The combination medicine according to any one of claims 1 to 6,
wherein the compound represented by General Formula [1] or the salt thereof is orally administered, and the pyrimidine antimetabolite is intravenously administered by drip infusion.

8. The combination medicine according to any one of claims 1 to 7,
wherein the combination medicine contains, as the pyrimidine antimetabolite, a compound selected from the group consisting of azacytidine, decitabine, guadecitabine, cytarabine, and gemcitabine, or a salt or hydrate thereof.

9. The combination medicine according to any one of claims 1 to 8,
wherein the combination medicine contains venetoclax or a salt thereof as the BCL-2 inhibitor.

10. The combination medicine according to any one of claims 1 to 8,
wherein the combination medicine contains, as the pyrimidine antimetabolite, decitabine or a salt or hydrate thereof, and a daily dose of the decitabine or the salt or hydrate thereof is 1 to 80 mg/m².

11. The combination medicine according to any one of claims 1 to 6 and 9,
wherein the compound represented by General Formula [1] or the salt thereof and the BCL-2 inhibitor are provided as a same composition.

12. The combination medicine according to claim 11,
wherein the combination medicine contains 0.3% to 50% by mass of the compound represented by General Formula [1] or the salt thereof and 40% to 99.7% by mass of the BCL-2 inhibitor with respect to a total amount of the combination medicine.

13. The combination medicine according to claim 11 or 12,
wherein the combination medicine is a tablet.

14. The combination medicine according to any one of claims 1 to 13,
wherein the combination medicine is used for treatment of hematological cancer.

15. The combination medicine according to any one of claims 1 to 14,
wherein the combination medicine is used for treatment of acute myeloid leukemia.
